**Europäisches Patentamt**

**European Patent Office**

**Office européen des brevets**

(19)

(11) Veröffentlichungsnummer: **0 009 619**
**B1**

(12) ## EUROPÄISCHE PATENTSCHRIFT

(45) Veröffentlichungstag der Patentschrift:
05.05.82

(51) Int. Cl.³: **A 61 N 1/04**

(21) Anmeldenummer: **79103182.6**

(22) Anmeldetag: **29.08.79**

(54) **Herzschrittmacherelektrode für transvenöse Anwendung.**

(30) Priorität: **30.09.78 DE 2843096**

(43) Veröffentlichungstag der Anmeldung:
**16.04.80 Patentblatt 80/8**

(45) Bekanntmachung des Hinweises auf die Patenterteilung:
**05.05.82 Patentblatt 82/18**

(84) Benannte Vertragsstaaten:
**AT BE CH DE FR GB IT NL SE**

(56) Entgegenhaltungen:

**DE-A-2 736 275**
**DE-A-2 149 449**
**FR-A-2 375 872**
**US-A-4 011 875**

**BIOMEDIZINISCHE TECHNIK, Band 24,**
**Nr. 1/2, 1. Februar 1979, Seiten 16-27**
**Berlin, DE.**
**H. J. BISPING: «Übersicht über verankerbare**
**transvenöse Schrittmachersonden»**

(73) Patentinhaber: **BIOTRONIK Mess- und Therapiegeräte**
**GmbH & Co Ingenieurbüro Berlin, Sieversufer 8,**
**D-1000 Berlin 47 (DE)**

(72) Erfinder: **Karr, Dieter E., Neheimer Strasse 4,**
**D-1000 Berlin 27 (DE)**
Erfinder: **Shanks, Scott B., 1305 Bonniebrae, Lake**
**Oswego, Oregon 97034 (US)**

(74) Vertreter: **Christiansen, Henning, Dipl.-Ing., Baseler**
**Strasse 172, D-1000 Berlin 45 (DE)**

Herzschrittmacherelektrode für transvenöse Anwendung

Die Erfindung betrifft eine Herzschrittmacherelektrode für transvenöse Anwendung mit einer Zuleitung und einer mit dieser in elektrischem Kontakt stehenden Drahtfederanordnung zur Befestigung im Herzen, die während des Einführungsvorgangs von einem Hohlzylinder aufgenommen wird, wobei die Drahtfederanordnung mit einem Kolben verbunden ist, der derart verschiebbar gelagert ist, dass durch den Druck eines innerhalb der Zuleitung geführten Mandrins auf den Kolben die Drahtfederanordnung aus dem Hohlzylinder austritt und ihre Position zum Eingriff in das Herzgewebe einnimmt.

Eine derartige Herzschrittmacherelektrode ist aus der DE-A-2 133 304 bekannt. Bei dieser Anordnung besteht der Nachteil, dass eine sichere Kontaktgabe zwischen der gewendelten Zuleitung und der die eigentliche Stimulation des Herzgewebes verursachenden, mit dem Kolben verbundenen Drahtfederanordnung im implantierten Zustand nicht sicher gewährleistet ist. Unterbrechungen der Kontaktgabe, die die Funktion des Herzschrittmachers — insbesondere bei der Steuerung durch vom Herzen abgeleitete Signale — beeinträchtigen und erhöhte Übergangswiderstände innerhalb der Elektrodenanordnung setzen die Spannung der übertragenen Impulse herab, so dass deren Wirkung vermindert und eine ordnungsgemässe Stimulation des Herzens nicht mehr gesichert ist. Bei einigen Typen von Schrittmachern kann ein derartiger erhöhter Übergangswiderstand durch eine Heraufsetzung der Ausgangsimpulsamplituden ausgeglichen werden.

Eine Folge davon ist jedoch ein erhöhter Energieverbrauch und damit eine verkürzte Lebensdauer der Energiequellen des Schrittmachers, so dass eine vorzeitige Neuimplantation erforderlich werden kann.

Bei der bekannten Elektrode liegen zwar die Drahtfederanordnung bildenden Drahtenden im ausgefahrenen und dabei aufgespreizten Zustand an dem elektrisch leitenden Hohlzylinder an, wenn sie entspannt sind. Da die Drahtenden zum Fixieren der Elektrode aber in das Herzgewebe einstechen, werden sie beim Eindringen in das Gewebe vorwiegend parallel geführt, so dass sie sich (unter Berücksichtigung der auf sie wirkenden Haltekräfte bei der Herzeigenbewegung) nur zeitweise in sicherem mechanischen und damit elektrischen Kontakt mit der Vorderkante des elektrisch leitenden Hohlzylinders befinden. Da auch die galvanische Verbindung über den verschiebbaren Kolben ihren Widerstand zeitabhängig verändern kann, besteht die Gefahr der Entstehung von Artefakten auf dem Übertragungsweg von Stimulationsort und Schrittmacher.

Der Erfindung liegt die Aufgabe zugrunde, eine Herzschrittmacherelektrode der obengenannten Gattung anzugeben, bei der für eine sichere Stimulation über die in das Gewebe eingreifenden Drahtenden eine sichere Kontaktgabe innerhalb der Anordnung während der gesamten Betriebsdauer des Schrittmachers gewährleistet ist.

Diese Aufgabe wird erfindungsgemäss mittels der im kennzeichnenden Teil des Hauptanspruchs angegebenen Massnahmen gelöst.

Durch diese kräftemässige Entkopplung des Kontaktelements von der Drahtfederanordnung ist gewährleistet, dass die für die Stimulation wirksame Fläche, die den elektrischen Kontakt zwischen Herzgewebe und Schrittmacher herstellt, unabhängig von auf die an der Befestigung der Elektrode auftretenden Belastungen im Betrieb des Schrittmachers im wesentlichen konstant ist und nicht durch nur zeitweise Kontaktgabe ein Teil dieser Fläche unregelmässig «abgeschaltet» wird. Das erfindungsgemässe, vorzugsweise eine Art «Schleifkontakt» bildende Kontaktelement liegt beständig an der Innenseite des Hohlzylinders mit nahezu konstanter Kraft federnd an.

Besonders vorteilhaft dabei ist, dass diese Kontaktgabe auch sichergestellt ist, wenn die Passung des Kolbens in dem Zylinder eine gewisse Toleranz beinhaltet, so dass die gesamte aus Drahtfederanordnung, Kolben und federnd anliegendem Element bestehende Anordnung leichter verschieblich ist als eine solche bei der die stetige elektrische Kontaktgabe zwischen Drahtenden und Hohlzylinder über eine entsprechend stramm eingepasste Kolbenwandung sichergestellt werden muss. Eine derartige Herabsetzung der Reibkraft beim Ausfahren der in das Herzgewebe eingreifenden Drahtfederanordnung ist deshalb von Bedeutung, weil die (gewendelte) Zuleitung aus besonders weichem Material hergestellt sein muss, damit die erforderliche Bruchsicherheit bei der grossen Zahl von im Betrieb aufgrund der Herztätigkeit und der Armbewegungen des Schrittmacherträgers zu ertragenden Biegebeanspruchungen gewährleistet ist. Bei einem zu grossen zum Ausfahren der Drahtfederanordnung durch den Führungsdraht (Mandrin) aufzubringenden Druck würde sich die weiche Zuleitungswendel in ihrer Lage verändern, so dass dadurch das Elektrodenende in einem anderen Bereich der Herzkammer bzw. des Vorhofs zu liegen kommen kann und damit die zuvor bei noch eingefahrenen Drahtenden durchgeführte Schwellenmessung zum Bestimmen des Fixierungsortes der Elektrode möglicherweise keine Gültigkeit mehr hat.

Bei einer vorteilhaften Weiterbildung der Erfindung bilden das Kontaktelement und die Drahtfederanordnung eine durch eine entsprechende in Längsrichtung verlaufende Öffnung des Kolbens hindurchgeführte Einheit, so dass durch die damit verbundene vereinfachte Herstellung eine Kostenersparnis erzielt werden kann. Günstig ist dabei auch, dass die federnden Längen der Anordnung zusammenhängend und in Längsrichtung durchgehend ausgebildet sein können, so dass sich für die gesamte Federanordnung bessere Dimensionierungsmöglichkeiten ergeben.

Der Öffnungsbereich des Kolbens wird zwecks Dichtung mit einer geeigneten elastischen Füllmasse, wie beispielsweise Silikonkautschuk verschlossen. Die im Silikonkautschuk verlaufende Länge der Drahtenden bzw. der Drahtfederanordnung steht damit trotzdem als federnder Bereich zur Verfügung.

Wenn das Kontaktelement bzw. der durch den Kolben hindurchgeführte Bereich der Drahtfederanordnung in seinem rückwärtigen Bereich, eine Öse bildet, die mittels eines an seinem Ende einen Haken aufweisenden Führungsdrahts ergriffen und zurückgezogen werden kann, ist der besondere Vorteil gegeben, dass — falls ein erster Befestigungsort der Elektrode im Herzen keine befriedigenden Stimulationsergebnisse liefert — ein Wiedereinfahren der Drahtfederanordnung in den Hohlzylinder zum unbehinderten Bewegen für eine Neulokalisation der Elektrode möglich ist. Wird nämlich die Elektrode mit ausgefahrener Drahtfederanordnung durch die Vene zurückgezogen, so besteht die Gefahr der Beschädigung von Vene und Herzklappen.

Das Ergreifen der Öse mit dem Haken des Führungsdrahts wird erleichtert, wenn das Ende des Führungsdrahts im Bereich des Hakens einen unrunden, beispielsweise abgeflachten oder mit einem Ansatz versehenen Querschnitt aufweist, so dass der behandelnde Arzt, nachdem er den Führungsdraht bis zum Anschlag innerhalb der Zuleitung vorangeschoben hat, durch Drehung des Mandrins und Erfühlen des Widerstandes, den dieser der Drehung entgegensetzt, die Position des Hakens in bezug auf die Öse feststellen kann. Der Haken des Führungsdrahts kann damit förmlich in die Öse einrasten und der Führungsdraht braucht dann zum Einfahren der Drahtenden nur noch so weit zurückbewegt zu werden bis das rückwärtige Ende der Öse das Ende der Zuleitung erreicht hat und dem weiteren Zurückziehen einen Widerstand entgegensetzt. Durch diese Mittel ist dem die Elektrode fixierenden Arzt ohne weiteres möglich, den Führungsdraht trotz der Kleinheit der Anordnung blind in die Öse einzuhaken.

Eine derartige an einem in einen Hohlzylinder geführten Kolben vorgesehene Öse ist unabhängig vom Vorhandensein eines die Kontaktgabe verbessernden «Schleifers» und unabhängig von der Gestaltung der in das Herzgewebe eingreifenden Drahtfederanordnung vorteilhaft anwendbar.

Es ist vorteilhaft, wenn der Kolben an der Innenseite des Hohlzylinders dichtend anliegt und damit eine Sperre gegen von aussen in das Innere des Hohlzylinders eindringende Körperflüssigkeit bildet. Wenn der Kolben beispielsweise sich in Richtung auf die Frontfläche der Elektrode verjüngend ausgebildet ist und ein Bereich der Innenfläche des Zylinders, der im ausgefahrenen Zustand der Drahtfederanordnung mit der Fläche des Kolbens in Kontakt kommt, entsprechend angepasst ist, so bildet er bei ausgefahrener Drahtfederanordnung im Betriebszustand der Elektrode eine Art Stopfen mit konischer Dichtfläche und besonders guter Dichtwirkung. Der Schutz des Inneren der Elektrode gegen eindringende Körperflüssigkeit sichert den elektrischen Kontakt zwischen der Innenwandung des Hohlzylinders und dem daran permanent anliegenden Kontaktelement.

Das Verhindern des Eindringens von Blut in den dem Herzen nahegelegenen Teil der wendelförmigen Zuleitung ist von besonderer Bedeutung, weil ein Koagulieren des Blutes in diesem Bereich zu einem Versteifen der Elektrodenzuleitung führen würde. Ein Versteifen würde aber eine Erhöhung des Kraftaufwandes zum Durchbiegen der Zuleitung bei jedem Herzschlag in dem Bereich der Zuleitung bedeuten, der am meisten von derartigen Verformungen betroffen ist. Der für die Biegung erforderliche vergrösserte Kraftaufwand überträgt sich als Zugwirkung auf die Befestigung der Elektrode im Herzen, die damit unverhältnismässig stark beansprucht wird, so dass eine Dislokation die Folge sein kann.

Besonders günstig ist dabei, dass die Art der verwendeten Dichtung nicht zur Erhöhung der Reibung beim Ausfahren der Drahtfederanordnung beiträgt, bei der Endstellung aber in äusserst zuverlässiger Weise eine Abdichtung gewährleistet.

Eine Vereinfachung und Verkürzung der Bauform der Elektrode lässt sich erreichen, wenn das Kontaktelement direkt die Innenseite der wendelförmigen Zuleitung — bei entsprechend gewähltem Durchmesser derselben — in Verlängerung des Hohlzylinders kontaktiert.

Eine vorteilhafte Ausführungsform der Erfindung einschliesslich einer Variante ist in den Zeichnungen dargestellt und wird nachfolgend näher beschrieben. Es zeigen:

Fig. 1 die Ausführungsform der erfindungsgemässen Herzschrittmacherelektrode in der Position zum Einführen durch die Vene

Fig. 2 die selbe Herzschrittmacherelektrode mit den Drahtenden in ausgefahrenem Zustand und

Fig. 3 und 4 verschiedene Querschnitte durch einen im Zusammenhang mit dieser Herzschrittmacherelektrode zu benutzenden Führungsdraht (Mandrin).

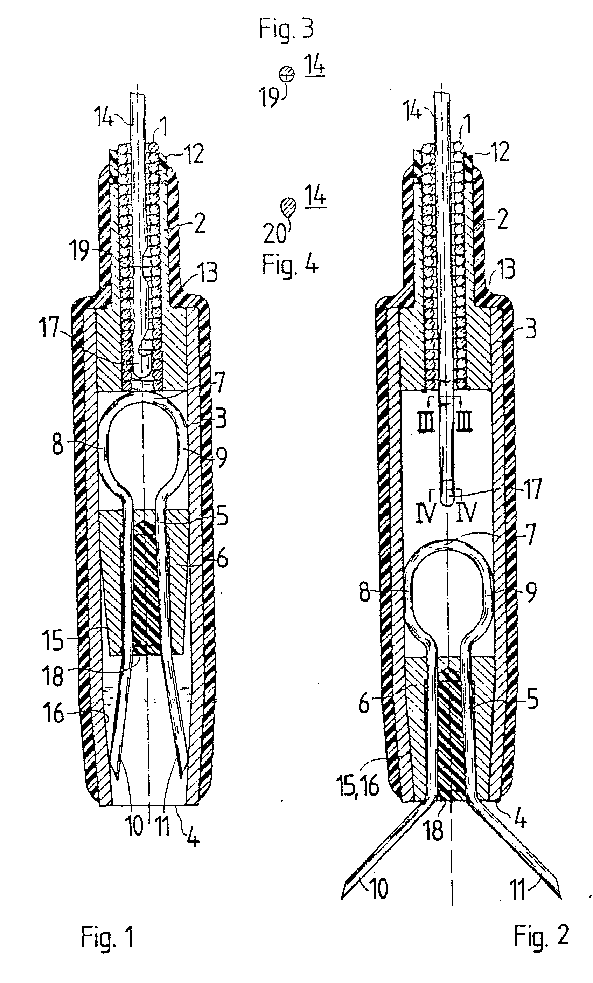

Bei der in Fig. 1 dargestellten Ausführungsform der erfindungsgemässen Elektrode ist die zum Herzschrittmacher führende und die elektrische Verbindung zu diesem herstellende gewendelte Zuleitung 1, die nur in ihrem dem Herzen nahegelegenen Bereich dargestellt ist, mit einer Hülse 2 versehen, die auf die Zuleitung aufgeschoben und gegebenenfalls durch Quetschen, Verschweissen oder dergleichen mit dieser verbunden ist. Auf die Hülse 2 ist ein Hohlzylinder 3 aufgeschoben und dort entsprechend befestigt, wobei dieser Hohlzylinder 3 wie die Hülse 2 aus leitendem Material besteht, so dass seine Frontfläche 4 geeignet ist, elektrische Stimulationsimpulse zum Herzgewebe zu übertragen. Der Hohlzylinder 3 umschliesst einen in Richtung zum herzseitigen Ende der Elektrodenanordnung offenen Hohlraum, in den eine Drahtfederanordnung 5 eingefügt ist. Diese ist ihrerseits mit einem Kolben 6 verbunden, der in dem Hohlzylinder 3 in Längsrichtung verschiebbar angeordnet ist.

Die Drahtfederanordnung 5 ist durch Öffnungen des Kolbens 6 hindurchgesteckt und geht an der dem herzseitigen Ende abgewandten Seite des Kolbens 6 befindlichen Seite in den ösenförmigen Teil 7 über, welcher an der Innenseite des Hohlzylinders 3 die federnd anliegenden Kontaktelemente 8 und 9 umfasst.

Am herzseitigen Ende der Drahtfederanordnung 5 sind zwei Drahtenden 10 und 11 vorgesehen, welche im fixierten Zustand der Elektrode in das Herzgewebe eingreifen. Eine Füllung 12 aus Silikonkautschuk füllt einen im Innern des Kolbens 6 gebildeten Hohlraum aus und lässt einerseits eine elastische Bewegung der Drahtenden 10 und 11 zu, wirkt aber andererseits dichtend, so dass Körperflüssigkeit nicht durch die für die Drahtfederanordnung 5 im Kolben 6 vorgesehenen Öffnungen hindurch in das Innere des Hohlzylinders 3 eintreten kann.

Die Elektrodenanordnung ist bis auf den Bereich der Frontfläche 4, die stimulierend wirkt, mit einer Isolierung umgeben, die im Bereich der gewendelten Zuleitung 1 als Isoliermantel 12 und im übrigen die Hülse 2 und den Hohlzylinder 3 umgebenden Bereich als Überzug 13 aus Silikonkautschuk ausgebildet ist. Als Werkstoff für die Metallteile kommen die bekannten körperverträglichen Materialien in Betracht, wie beispielsweise die unter den Bezeichnungen Elgiloy und Protasul bekannten Legierungen. Für die Auswahl des Werkstoffs für die Drahtfederanordnung 5 sind die für die im folgenden beschriebenen Funktionen notwendigen Elastizitätseigenschaften zu berücksichtigen.

Ein beim Einführen der Elektrode durch die Vene zur Versteifung der Elektrodenzuleitung dienender Mandrin 14 dient bei der dargestellten Ausführungsform gleichzeitig zum Aus- und Einfahren der Drahtenden 10 und 11 wie es ebenfalls nachfolgend im einzelnen beschrieben ist.

Während des Einführungsvorgangs befindet sich die Drahtfederanordnung 5 mitsamt den Drahtenden 10 und 11 in ihrer in den Hohlzylinder 3 zurückgeschobenen Stellung, so dass die Drahtenden 10 und 11 nicht vorzeitig mit dem Gewebe in Eingriff kommen können. Wenn nach Ausführung einer Reizschwellenmessung über die Frontfläche 4 eine günstige Position zur Befestigung der Elektrode in Vorhof oder Ventrikel gefunden wurde, wird vom entgegengesetzten Ende der Elektrodenzuleitung her Druck auf den Mandrin 14 ausgeübt. Dieser bewegt sich an dem ösenförmigen Teil 7 vorbei und übt in bekannter Weise auf die rückwärtige Fläche des Kolbens 6 eine Kraft aus, so dass bei festgehaltener Zuleitung 1 die Drahtfederanordnung 5 mitsamt dem Kolben 6 in Richtung auf das Herzgewebe verschiebbar ist. Sobald die Drahtenden 10 und 11 durch die Öffnung im Hohlzylinder 3 über die Frontfläche 4 hinausgelangen, haben sie das Bestreben, sich aufgrund ihrer Federvorspannung aufzuspreizen und eine Position einzunehmen, wie sie in Fig. 2 dargestellt ist.

In Fig. 2 ist die Drahtfederanordnung 5 in vollständig ausgefahrenem Zustand wiedergegeben. Wichtig ist dabei, dass der Kolben 6 soweit voranschiebbar ist bis seine konische Aussenfläche 15 (Fig. 1) an der angepassten Innenfläche des sich verjüngenden Bereiches 16 des Hohlzylinders anliegt. Infolge der sich ergebenden Dichtwirkung zwischen Kolben 6 und Hohlzylinder 3 kann keine die elektrische Kontaktwirkung möglicherweise beeinträchtigende Körperflüssigkeit in den hinter dem Kolben 6 befindlichen Raum und in den von der gewendelten Zuleitung 1 umschlossenen Bereich eindringen. Die seitlichen, die federnd anliegenden Elemente 8 und 9 bildenden Bereiche der Drahtfederanordnung 5 liegen beständig fest an der Innenwandung des Hohlzylinders 3 an, so dass eine sichere Kontaktgabe gewährleistet ist, wobei eine durch die beim Befestigen oder Halten der Elektrode im Körpergewebe auftretende Verbiegung der Drahtenden 10 und 11 nach innen eher noch eine Verstärkung der Andruckkraft bewirkt.

Die Federwirkung trägt auch dazu bei, dass der Kolben 6 nicht zu leichtgängig verschiebbar ist, wobei die Fixierung in der Endlage allerdings im wesentlichen durch «Stopfenwirkung» der aufeinanderliegenden, die Dichtung bildenden und sich verjüngenden Bereiche, sowie die auf die Drahtenden 10 und 11 ausgeübte Zugkraft bewirkt wird — nachdem sich diese im Herzgewebe verfangen haben.

Eine aus Silikonkautschuk bestehende Füllung 18 des Kolbens 6 verschliesst einerseits die Öffnungen in der Stirnseite, durch die die Drahtfederanordnung 5 hindurchtritt, gegen eindringende Körperflüssigkeit und sorgt andererseits für einen festen Sitz derselben, ohne die Biegsamkeit der Drahtenden einzuschränken.

Falls die erste Fixierung der Elektrode nicht zufriedenstellend erfolgt ist, kann eine Befestigung an anderer Stelle im Herzen vorgenommen werden, ohne dass es erforderlich ist, die Elektrode vollständig aus der Vene herauszuziehen, damit der Kolben in seine Ausgangslage gebracht werden und so die Drahtenden in den Hohlzylinder 3 zurückgezogen werden können. Ein derartiges Zurückziehen der Elektrode mit zum Verhaken ausgefahrenen Drahtenden würde zudem eine Gefährdung von Herzklappen und Veneninnerem bedeuten.

Dadurch, dass bei der erfindungsgemässen Ausführungsform der Mandrin 14 (der in Fig. 2 gegenüber Fig. 1 um 90° gedreht dargestellt ist) einen hakenförmigen Teil 17 aufweist, besteht die Möglichkeit, letzteren in den ösenförmigen Teil 7 einzuhaken und durch Zurückziehen des Mandrins bei festgehaltener Zuleitung die Drahtfederanordnung 5 mit dem Kolben 6 in den Hohlzylinder 3 zurückzuziehen, so dass sich wieder der in Fig. 1 dargestellte Zustand ergibt. Damit ist es möglich, die Elektrode in der Vene ohne Gefährdung von Organen vor- und zurückzuschieben. Eine Erleichterung für das Einhaken des Mandrins 14 in den ösenförmigen Teil 7 zum Zurückziehen der Drahtfederanordnung 5 ergibt sich, wenn der hakenförmige Teil 17 des Mandrins als Abflachung 19 oder Nase 20 ausgebildet ist, d.h. einen unrunden Querschnitt aufweist, wie es in den Fig. 3 und 4 dargestellt ist.

Damit wird einer Drehung des Mandrins innerhalb der Zuleitung ein unterschiedlicher Widerstand entgegengesetzt, wenn der hakenförmige Teil sich im Bereich der Öse befindet und an dieser seitlich anliegt. Auf diese Weise ist für den Arzt eine wirksame Möglichkeit gegeben, die Position des hakenförmigen Teils in bezug auf die Öse schnell und zuverlässig blind zu ertasten, um die beabsichtigten Manipulationen der Elektrode ohne grosse Verzögerung ausführen zu können. Liegt die einen Ansatz bildende Nase 20 an der Öse an, so ist die Richtung des Hakens durch einen erhöhten Widerstand beim Drehen des Mandrins 14 kenntlich. Das Ergreifen der Öse durch den hakenförmigen Teil 17 wird auch dadurch erleichtert, dass der Mandrin zum Ergreifen der Öse zunächst bis zu dem durch die rückwärtige Fläche des Kolbens 6 gebildeten Anschlag geführt werden kann. In diesem Fall liegt der Bereich mit der Abflachung 19 an der Öse an und macht die Richtung des Hakens durch eine gewisse Rastwirkung beim Drehen des Mandrins 14 deutlich. Durch die nach dem Zurückziehen aus dieser Position auftretenden Kraftverhältnisse ist es dem Arzt ohne weiteres möglich, zu beurteilen, ob der Haken in den ösenförmigen Teil eingegriffen hat. (Für diese Funktion ist es dabei ausreichend, wenn nur entweder die Nase 19 oder die Abflachung 20 vorgesehen ist.)

Auch für die Gestaltung der federnd anliegenden Elemente sind im Rahmen des Umfangs der Erfindung vielfältige Gestaltungs- und Ausführungsmöglichkeiten denkbar. So kann der ösenförmige Teil bei einem entsprechenden Durchmesser der gewendelten Zuleitung auch innerhalb dieser gleiten, so dass der Hohlzylinder 3 verkürzt bzw. gegebenenfalls ganz auf diesen verzichtet werden kann.

**Patentansprüche**

1. Herzschrittmacherelektrode für transvenöse Anwendung mit einer Zuleitung (1) und einer mit dieser in elektrischem Kontakt stehenden Drahtfederanordnung (5) zur Befestigung der Elektrode im Herzen, die während des Einführungsvorgangs zum Schutz des Körpergewebes von einem Hohlzylinder (3) aufgenommen wird, wobei die Drahtfederanordnung mit einem Kolben (6) verbunden ist, der derart verschiebbar gelagert ist, dass durch den Druck eines innerhalb der Zuleitung geführten Mandrins (14) auf den Kolben die Drahtfederanordnung aus dem Hohlzylinder austritt, gekennzeichnet durch mindestens ein mit der Drahtfederanordnung (5) elektrisch leitend verbundenes, federndes Kontaktelement (8, 9), welches derart ausgebildet ist, dass es an einem mit der Zuleitung (1) fest verbundenen, elektrisch leitenden Bereich der Innenwandung des Hohlzylinders (3) bei im wesentlichen punkt- oder linienförmiger Kontaktgabe unabhängig von auf die Drahtfederanordnung von aussen ausgeübten Kräften permanent anliegt.

2. Herzschrittmacherelektrode nach Anspruch 1, dadurch gekennzeichnet, dass das Kontaktelement (8, 9) schleiferartig ausgebildet ist.

3. Herzschrittmacherelektrode nach einem der Ansprüche 1 oder 2, dadurch gekennzeichnet, dass das Kontaktelement (8, 9) in einem gegen Körperflüssigkeit durch den Kolben (6) dichtend abgeschlossenen Bereich des Hohlzylinders (3) angeordnet ist und die elektrische Verbindung mit der Drahtfederanordnung durch den Kolben (6) hindurch erfolgt.

4. Herzschrittmacherelektrode nach Anspruch 3, dadurch gekennzeichnet, dass eine Öffnung des Kolbens (6), durch die die elektrische Verbindung zwischen der Drahtfederanordnung (5) und dem Kontaktelement (8, 9) hindurchgeführt ist, mit einer Füllung (18) aus dauerelastischem Material verschlossen ist.

5. Herzschrittmacherelektrode nach einem der Ansprüche 3 oder 4, dadurch gekennzeichnet, dass das Kontaktelement (8, 9) und die Drahtfederanordnung (5) einstückig ausgebildet sind.

6. Herzschrittmacherelektrode nach einem der Ansprüche 3 bis 5, dadurch gekennzeichnet, dass der Aussendurchmesser des Kolbens (6) sich in Richtung auf seine dem Frontende der Elektrode zugewandte Stirnfläche hin verjüngt (konische Aussenfläche 15), so dass er im ausgefahrenen Zustand der Drahtfederanordnung (5) an einer entsprechend ausgebildeten Gegenfläche (Bereich 16) des Hohlzylinders (3) gegen Körperflüssigkeit dichtend anliegt.

7. Herzschrittmacherelektrode nach einem der Ansprüche 1 bis 6, dadurch gekennzeichnet, dass der Kolben (6) an seinem rückwärtigen Ende einen ösenförmigen Teil (7) aufweist, der mit einem hakenförmigen Teil (17) eines Führungsdrahts (14) derart in Eingriff gebracht werden kann, dass die Drahtfederanordnung (5) in den Hohlzylinder (3) zurückziehbar ist.

8. Herzschrittmacherelektrode nach Anspruch 7, dadurch gekennzeichnet, dass der ösenförmige Teil (7) durch einen rückwärtigen Teil der Drahtfederanordnung (5) und/oder des Kontaktelements (8, 9) gebildet wird.

9. Führungsdraht für eine Herzschrittmacherelektrode nach einem der Ansprüche 7 oder 8, dadurch gekennzeichnet, dass er im Bereich seines hakenförmigen Teils (17) eine Abflachung (19) des Querschnitts oder einen Ansatz (20) aufweist, wobei die Abflachung (19) bzw. der Ansatz (20) in Umfangsrichtung eine definierte Position in bezug auf den hakenförmigen Teil (17) einnimmt.

**Claims**

1. A pacemaker electrode for transvenous application with a supply line (1) and a spring wire arrangement (5) which is in electrical contact therewith, for fixing the electrode in the heart, said electrode being housed in a hollow cylinder (3) during insertion in order to protect the body tissue, and the spring wire arrangement being connected to a plunger (6) which is mounted so as to be displaceable such that, due to the pressure on the plunger of a mandrin which is guided within the supply line, the spring wire arrangement passes out of the hollow cylinder, characterised by at least one resilient contact element (8, 9) which is connected electrically to the spring wire arrangement (5) and is so formed that it permanently abuts an electrically conductive region of the inner walls of the hollow cylinder (3) via an essentially punctiform or line form contact independently of external forces applied to the spring wire arrangement, said region being fixedly connected to the supply line (1).

2. A pacemaker electrode according to claim 1, characterised in that the contact element (8, 9) is in loop form.

3. A pacemaker electrode according to claim 1 or 2, characterised in that the contact element (8, 9) is arranged in a region of the hollow cylinder (3) which is hermetically sealed by the plunger (6) against body fluid and the electrical connection with the spring wire arrangement is made through the plunger (6).

4. A pacemaker electrode according to claim 3, characterised in that an opening in the plunger (6), through which the electrical connection is passed between the spring wire arrangement (5) and the contact element (8, 9) is closed off by a filling (18) made of permanently elastic material.

5. A pacemaker electrode according to claim 3 or 4, characterised in that the contact element (8, 9) and the spring wire arrangement (5) are integral.

6. A pacemaker electrode according to any one of claims 3 to 5, characterised in that the outer diameter of the plunger (6) tapers (conical outer face 15) towards its end facing the front end of the electrode, so that, when the spring wire arrangement has passed out, the plunger abuts a mating face (region 16) of the hollow cylinder (3) which is formed accordingly so as to provide a seal against body fluid.

7. A pacemaker electrode according to any one of claims 1 to 6, characterised in that the plunger (6) has an eyelet type member (7) on its rear end, said eyelet type member being capable of engagement with a hook-shaped portion (17) of a guide wire (14) such that the spring wire arrangement (5) can be withdrawn into the hollow cylinder (3).

8. A pacemaker electrode according to claim 7, characterised in that the eyelet shaped member (7) is formed by a rear member of the spring wire arrangement (5) and/or the contact element (8, 9).

9. A guide wire for a pacemaker electrode according to claim 7 or 8, characterised in that in the region of its hook-shaped portion it has a flattened portion (19) of its cross section or an extension (20) and the flattened portion (19) or the extension (20) occupies a predetermined circumferential position relative to the hook-shaped portion (17).

**Revendications**

1. Electrode de stimulateur cardiaque pour application transveineuse, comprenant une connexion (1) et un dispositif à ressort en fil (5) en contact électrique avec la connexion, servant à la fixation de l'électrode dans le coeur et reçu par un cylindre creux (3) pendant l'opération d'introduction, le dispositif à ressort en fil étant relié à un piston (6) monté coulissant de telle manière qu'une poussée exercée sur le piston par un mandrin (14) guidé dans la connexion fait sortir le dispositif à ressort en fil partiellement hors du cylindre creux, caractérisée par au moins un élément de contact élastique (8, 9) relié électriquement au dispositif à ressort en fil (5), qui est réalisé de telle manière qu'il s'applique en permanence contre une portion conductrice électrique reliée solidement à la connexion (1) de la paroi interne du cylindre creux (3), en établissant avec elle un contact essentiellement ponctuel ou linéaire, indépendamment des forces exercées de l'extérieur sur le dispositif à ressort en fil.

2. Electrode de stimulateur cardiaque selon la revendication 1, caractérisée en ce que l'élément de contact (9, 8) est réalisé à la façon d'un frotteur.

3. Electrode de stimulateur cardiaque selon la revendication 1 ou 2, caractérisée en ce que l'élément de contact (8, 9) est disposé dans une partie du cylindre creux (3) fermée de façon étanche par le piston (6) contre la pénétration de liquide corporel et en ce que la liaison électrique avec le dispositif à ressort en fil passe par le piston (6).

4. Electrode de stimulateur cardiaque selon la revendication 3, caractérisée en ce qu'une ouverture du piston (6) par laquelle passe la liaison électrique entre le dispositif à ressort en fil (5) et l'élément de contact (8, 9) est fermée par un remplissage (18) d'une matière qui reste élastique.

5. Electrode de stimulateur cardiaque selon la revendication 3 ou 4, caractérisée en ce que l'élément de contact (8, 9) et le dispositif à ressort en fil (5) sont d'un seul tenant.

6. Electrode de stimulateur cardiaque selon l'une des revendications 3 à 5, caractérisée en ce que le diamètre extérieur du piston (6) se rétrécit en direction de sa face extrême dirigée vers l'extrémité frontale de l'électrode (surface extérieure conique 15), de sorte que le piston, à l'état étendu du dispositif à ressort en fil (5), s'applique de façon étanche contre une surface antagoniste (partie 16) complémentaire du cylindre creux (3) en empêchant la pénétration de liquide corporel.

7. Electrode de stimulateur cardiaque selon l'une des revendications 1 à 6, caractérisée en ce que le piston (6) présente, à son extrémité postérieure, une partie en forme de boucle (7) qui peut être reliée de telle manière à une partie (17) en forme de crochet d'un fil de guidage (14) que le dispositif à ressort en fil (5) est rétractable dans le cylindre creux (3).

8. Electrode de stimulateur cardiaque selon la revendication 7, caractérisée en ce que la partie (7) en forme de boucle est formée par une partie postérieure du dispositif à ressort en fil (5) et/ou de l'élément de contact (8, 9).

9. Fil de guidage pour une électrode stimulateur cardiaque selon la revendication 7 ou 8, caractérisé en ce qu'il présente, dans la région de sa partie (17) en forme de crochet, un méplat (19) en section ou une protubérance (20), le méplat (19) ou la protubérance (20) occupant une position définie dans le sens circonférenciel par rapport à la partie (17) en forme de crochet.

Fig. 3

$\underline{14}$

19 $\oplus$

$\underline{14}$

20 $\otimes$

Fig. 4

Fig. 1

Fig. 2